(19) **Europäisches Patentamt / European Patent Office / Office européen des brevets**

(11) **EP 1 835 968 B2**

(12) # NEW EUROPEAN PATENT SPECIFICATION
### After opposition procedure

(45) Date of publication and mention
of the opposition decision:
**01.04.2015 Bulletin 2015/14**

(45) Mention of the grant of the patent:
**18.08.2010 Bulletin 2010/33**

(21) Application number: **05854884.3**

(22) Date of filing: **21.12.2005**

(51) Int Cl.:
*A61Q 11/02* (2006.01)    *A61K 8/22* (2006.01)
*A61K 8/81* (2006.01)

(86) International application number:
**PCT/US2005/046240**

(87) International publication number:
**WO 2006/073822 (13.07.2006 Gazette 2006/28)**

(54) **TOOTH WHITENING COMPOSITION CONTAINING CROSS-LINKED POLYMER-PEROXIDES**

ZAHNBLEICHZUSAMMENSETZUNG MIT VERNETZTEN POLYMER-PEROXIDEN

COMPOSITION DE BLANCHIMENT DES DENTS CONTENANT UN POLYMERE RETICULE ET DES PEROXYDES

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.12.2004 US 640656 P**
**23.11.2005 US 285871**

(43) Date of publication of application:
**26.09.2007 Bulletin 2007/39**

(73) Proprietor: **Colgate-Palmolive Company**
**New York NY 10022-7499 (US)**

(72) Inventors:
• **SHASTRY, Ramachandra**
**Dayton, NJ 08810 (US)**
• **MIRAJKAR, Yelloji, Rao**
**Piscataway, NJ 08854 (US)**
• **DIXIT, Nagaraj**
**Plainsboro, NJ 08536 (US)**
• **CAMERON, Ryan**
**Somerset, NJ 08873 (US)**
• **WANG, Qin**
**Monmouth Junction, NJ 08852 (US)**
• **ZAIDEL, Lynette**
**Cranford, NJ 07016 (US)**
• **CHOPRA, Suman, K.**
**Dayton, NJ 08810 (US)**
• **PRENCIPE, Michael**
**West Windsor, NJ 08550 (US)**

(74) Representative: **Jenkins, Peter David et al**
**Page White & Farrer**
**Bedford House**
**John Street**
**London WC1N 2BF (GB)**

(56) References cited:
EP-A- 0 417 971          WO-A-01/51012
WO-A-91/07184            WO-A-2005/070378
WO-A-2006/026424         WO-A1-98/16206
WO-A2-2004/071323        GB-A- 1 205 325
US-A- 5 145 675          US-A1- 2003 211 440
US-A1- 2004 086 468      US-A1- 2004 105 834

• Amcol Health and Beauty solutions (cited in third party observations during prosecution of the opposed patent as D5)
• Evidence that Poly-Pore is a delivery system
• Evidence that magnabrite and Polargel are rheology modifiers
• Wikipedia - High-test peroxide
• 'Collins English Dictionary', vol. 1, HARPERCOLLINS PUBLISHERS, GLASGOW page 330
• Eudragit L100-55 data sheet
• K. EMBIL ET AL.: 'The Microsponge® Delivery System (MDS): a topical delivery system with reduced irritancy incorporating multiple triggering mechanisms for the release of actives' J. MICROENCAPSULATION vol. 13, no. 5, 1996, pages 575 - 588

**Description**

BACKGROUND OF THE INVENTION

Stained, yellowed or discolored teeth are almost inevitable due to the absorbent nature of dental material. Everyday activities such as smoking or other oral use of tobacco products, and eating, chewing or drinking certain foods and beverages (in particular coffee, tea and red wine) cause undesirable staining of surfaces of teeth. Staining can also result from microbial activity, including that associated with dental plaque. Even with regular brushing and flossing, years of chromogen accumulation can impart noticeable tooth discoloration.

Professional dental treatments frequently include a tooth surface preparation such as acid etching followed by the application of highly concentrated bleaching solutions (e.g., up to 37% hydrogen peroxide) and/or the application of heat or light. These procedures provide rapid results, but are expensive, and often require several trips to the dentist. In many cases, the patient's lips are uncomfortably retracted during the entire treatment and the patient is confined to sitting in the dental chair.

Alternatively, at home bleaching systems can be used. These systems have gained significant popularity in the past decade because of reduced cost, and increased convenience. Instead of time consuming and frequent trips to the dentist, the tooth whitener is purchased at a consumer retail store and may be used while performing other personal tasks or errands, relaxing or sleeping.

Current home treatment methods include abrasive toothpastes, toothpastes that produce oxides, whitening gels for use with a dental tray and whitening strips. The effectiveness of such techniques depends on a variety of factors including the type and intensity of the stain, the type of bleaching agent, contact time of the bleaching agent on the teeth, the amount of available bleaching active in the composition, the ability of the bleaching agent to penetrate the tooth enamel, and consumer compliance. Effectiveness is also dependant on the amount of bleaching active in the composition, the ability of the active to be released during use, and the stability of the active in the product. However, the effectiveness of many of these treatments is adversely affected because of deficiencies in one or more factors relating to the composition and consumer compliance.

[0005a] WO-A-91/07184 discloses substantially anhydrous complexes of PVP (polyvinylpyrrolidone) and $H_2O_2$ in molar ratios of between about 2:1 and 1:1 respectively (corresponding to between about 13% and about 23% by weight $H_2O_2$). The complexes are substantially uniform, free-flowing white powders.

[0005b] US-A-2004/0086468 discloses a delivery system for delivering a tooth whitening substance to a surface of an oral cavity, comprising a film of flexible malleable polymer material having a tooth whitening substance as an integral component of the film. Again, the polymer of the polymer - $H_2O_2$ complex is a PVP polymer.

[0005c] WO-A-2005/070378 comprises part of the state of the art under Article 54(3)-EPC and discloses an anhydrous liquid tooth whitening composition comprising a peroxide-containing compound and an orally acceptable carrier which includes a humectant, a bioadhesive agent and a film-forming agent. Various polymers are disclosed for use in the peroxide-containing compound.

[0005d] WO-A-2006/026424 comprises part of the state of the art under Article 54(3) EPC. This document discloses an oral care composition comprising (a) a peroxide complex comprising hydrogen peroxide and an N-vinyl heterocyclic polymer; (b) a whitening agent; and (c) an orally acceptable carrier. The carrier may comprise a film-forming material.

BRIEF SUMMARY OF THE INVENTION

The present invention provides an oral care composition according to claim 1. The present invention provides an oral care composition according to claim 12.

The present invention also provides a method for whitening a tooth surface according to claim 16.

DETAILED DESCRIPTION OF THE INVENTION

It has been discovered that compositions and methods of this invention may afford advantages over oral care compositions among known in the art, including one or more of enhanced whitening efficacy, provision of a higher available concentration of bleaching agent, tooth adherence in the presence of saliva without the use of a dental tray, and sustained and/or controlled release of the bleaching agent.

The present invention provides oral care compositions and methods for administration or application to, or use with, a human or other animal subject.

The present invention provides compositions comprising a peroxide composite. The peroxide composite is preferably present at a level of 0.5% to 60%, optionally 1% to 50%, optionally 5% to 40%, optionally 10% to 25%.

The peroxide composite may include a peroxide compound and a porous cross-linked polymer. As referred to herein, a "peroxide compound" is an oxidizing compound comprising a bivalent oxygen-oxygen group. Peroxide compounds include peroxides and hydroperoxides, such as hydrogen peroxide, peroxides of alkali and alkaline earth metals, organic peroxy compounds, peroxy acids, pharmaceutically-acceptable salts thereof, and mixtures thereof. Peroxides of alkali and alkaline earth metals include lithium peroxide, potassium peroxide, sodium peroxide, magnesium peroxide, calcium peroxide, barium peroxide, and mixtures thereof. Organic peroxy compounds include carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as persulfate, dipersulfate, percarbonate, perphosphate, perborate and persilicate salts of alkali and alkaline earth metals such as lithium, potassium, sodium, magnesium, calcium and barium, and mixtures thereof. In various embodiments, the peroxide compound comprises hydrogen peroxide, urea peroxide, sodium percarbonate and mixtures thereof. In one embodiment, the peroxide compound comprises hydrogen peroxide. In one embodiment, the peroxide compound consists essentially of hydrogen peroxide.

The peroxide composite comprises a porous cross-linked polymer. As referred to herein, a "porous cross-linked polymer" is a particulate polymer material which is operable to sorb a peroxide compound, The term "sorb" refers to the "sorptive" (or "sorption") capability of the polymer particles to adsorb, absorb, complex, or otherwise retain a peroxide compound. As used herein, "porous" refers to the presence of voids or interstices between cross-linked polymers that increases the overall surface area of the polymer beyond a solely perimeter measurement. Without limiting the mechanism, function or utility of the present invention, it is understood in some embodiments that the composite comprises polymeric particulates having a non-smooth surface and an irregular polymeric matrix in which the peroxide compound is retained. These chemical and physical characteristics of the particulate hinder the release of the peroxide compound from the polymer particulates, and in some embodiments provides sustained release of the peroxide compound.

In the oral care composition of claim 1, the cross-linked polymer has a BET (Brunauer, Emmett and Teller method) pore volume of 0.1 to 0.3 cc/g, optionally 0.1 to 0.2 cc/g, optionally 0.14 to 0.16 cc/g. In the oral care compositions of claims 1 and 12 and in the peroxide composite used in the method of claim 16, the peroxide composite comprises a peroxide compound at a level of 70 to 90% by weight of the peroxide composite.

The cross-linked polymer is preferably the polymerization product of at least one and preferably at least two monomers having at least two unsaturated bonds (hereinafter referred to as "polyunsaturated" monomers), the monomers being polymerized including no more than about 40% by weight, preferably less than about 9% by total monomer weight, of monounsaturated comonomers. The polyunsaturated monomers are selected from polyacrylates, polymethacrylates, polyitaconates and mixtures thereof. Included are poly-acrylates, -methacrylates, or -itaconates of: ethylene glycol, propylene glycol; di-, tri-, tetra-, or poly-ethylene glycol and propylene glycol; trimethylol propane, glycerin, erythritol, xylitol, pentaerythritol, dipentaerythritol, sorbitol, mannitol, glucose, sucrose, cellulose, hydroxyl cellulose, methyl cellulose, 1,2 or 1,3 propanediol, 1,3 or 1,4 butanediol, 1,6 hexanediol, 1,8 octanediol, cyclohexanediol, cyclohexanetriol, and mixtures thereof. Similarly, bis(acrylamido or methacrylamido) compounds can be used. These compounds are, for example, methylene bis(acryl or methacryl)amide, 1,2 dihydroxy ethylene bis(acryl or methacryl)amide, hexamethylene bis(acryl or methacryl)amide. In a preferred embodiment, the polyunsaturated monomer is polymethacrylate.

Another group of monomers that may be suitably utilized in the invention includes di or poly vinyl esters, such as divinyl propylene urea, divinyl-oxalate, -malonate, - succinate, -glutamate, -adipate, -sebacate, -maleate, -fumerate, -citraconate, and -mesaconate. Other suitable polyunsaturated monomers include divinyl benzene, divinyl toluene, diallyl tartrate, allyl pyruvate, allyl maleate, divinyl tartrate, triallyl melamine, N,N'-methylene bis acrylamide, glycerine dimethacrylate, glycerine trimethacrylate, diallyl maleate, divinyl ether, diallyl monoethyleneglycol citrate, ethyleneglycol vinyl allyl citrate, allyl vinyl maleate, diallyl itaconate, ethyleneglycol diester of itaconic acid, divinyl sulfone, hexahydro 1,3,5-triacryltriazine, triallyl phosphite, diallyl ether of benzene phosphonic acid, maleic anhydride triethylene glycol polyester, polyallyl sucrose, polyallyl glucose, sucrose diacrylate, glucose dimethacrylate, pentaerythritol di-, tri- and tetra-acrylate or methacrylate, trimethylol propane di- and triacrylate or methacrylate, sorbitol dimethacrylate, 2-(1-aziridinyl)-ethyl methacrylate, tri/ethanolamine diacrylate or dimethacrylate, triethanolamine triacrylate or trimethacrylate, tartaric acid dimethacrylate, triethyleneglycol dimethacrylate, the dimethacrylate of bis-hydroxy ethylacetamide and mixtures thereof.

Other suitable polyethylenically unsaturated cross-linking monomers include ethylene glycol diacrylate, diallyl phthalate, trimethylolpropanetrimethacrylate, polyvinyl and polyallyl ethers of ethylene glycol, of glycerol, of pentaerythritol, of diethyleneglycol, of monothio- and dithio-derivatives of glycols, and of resorcinol; divinylketone, divinylsulfide, allyl acrylate, diallyl fumarate, diallyl succinate, diallyl carbonate, diallyl malonate, diallyl oxalate, diallyl adipate, diallyl sebacate, diallyl tartrate, diallyl silicate, triallyl tricarballylate, triallyl aconitrate, triallyl citrate, triallyl phosphate, divinyl naphthalene, divinylbenzene, trivinylbenzene; alkyldivinylbenzenes having from 1 to 4 alkyl groups of 1 to 2 carbon atoms substituted on the benzene nucleus; alkyltrivinylbenzenes having 1 to 3 alkyl groups of 1 to 2 carbon atoms substituted on the benzene nucleus; trivinylnaphthalenes, polyvinylanthracenes, and mixtures thereof. In addition, acryl or methacryl-encapped siloxanes and polysiloxanes, methacryloyl end-capped urethanes, urethane acrylates of polysiloxane alcohols and bisphenol A bis methacrylate and ethoxylated bisphenol A bis methacrylate also are suitable as polyunsaturated monomers.

Still another group of monomers is represented by di or poly vinyl ethers of ethylene, propylene, butylene, glycols, glycerine, penta erythritol, sorbitol, di or poly allyl compounds such as those based on glycols, glycerine, or combinations of vinyl allyl or vinyl acryloyl compounds such as vinyl methacrylate, vinyl acrylate, allyl methacrylate, allyl acrylate, methallyl methacrylate, or methallyl acrylate. In addition, aromatic, cycloaliphatic and heterocyclic compounds are suitable for this invention. These compounds include divinyl benzene, divinyl toluene, divinyl diphenyl, divinyl cyclohexane, trivinyl benzene, divinyl pyridine, and divinyl piperidine. Furthermore, divinyl ethylene or divinyl propylene urea and similar compounds may be used, e.g., as described in U.S. Patents 3,759,880; 3,992,562; and 4,013,825. Acryloyl- or methacryloyl end-capped siloxane and polysiloxanes such as those described in U.S. Patent 4,276,402; 4,341,889, may be suitable for this invention. Methacryloyl end-capped urethanes, such as those described in U.S. Patents 4,224,427; 4,250,322; and 4,423,099, may be suitable for this invention. Urethane acrylates of polysiloxane alcohols as described in U.S. Patents 4,543,398 and 4,136,250, and bisphenol A bis methacrylate and ethoxylated bisphenol A bis methacrylate may also be suitable monomers for this invention.

Monoethylenically unsaturated monomers may also be suitable, in an amount up to about 40% by weight, preferably no more than about 9% by weight, based on the total weight of monomers, for preparing polymer micro-particles include ethylene, propylene, isobutylene, disobutylene, styrene, vinyl pyridine ethylvinylbenzene, vinyltoluene, and dicyclopentadiene; esters of acrylic and methacrylic acid, including the methyl, ethyl, propyl, isopropyl, butyl, sec-butyl, tert-butyl, amyl, hexyl, octyl, ethylhexyl, decyl, dodecyl, cyclohexyl, isobornyl, phenyl, benzyl, alkylphenyl, ethoxymethyl, ethoxyethyl, ethoxyproyl, propoxymethyl, propoxyethyl, propoxypropyl, ethoxyphenyl, ethoxybenzyl, and ethoxycyclohexyl esters; vinyl esters, including vinyl acetate, vinyl propionate, vinyl butyrate and vinyl laurate, vinyl ketones, including vinyl methyl ketone, vinyl ethyl ketone, vinyl isopropyl ketone, and methyl isopropenyl ketone, vinyl ethers, including vinyl methyl ether, vinyl ethyl ether, vinyl propyl ether, and vinyl isobutyl ether.

Other monounsaturated monomer materials which may be utilized in accordance with the present invention, in an amount up to about 40% by weight or less, preferably no more than about 25% by weight, and most preferably no more than about 9% by weight, based on the total weight of monomers in the monomer solution, include hydroxy alkyl esters of alpha, beta-unsaturated carboxylic acids such as 2-hydroxy ethylacrylate or methacrylate, hydroxypropylacrylate or methacrylate. Many derivatives of acrylic or methacrylic acid other than the esters mentioned are also suitable as starting monounsaturated monomer materials for use in forming the unsaturated polymer micro-particles of the present invention. These include, but are not limited to the following monomers: methacrylylglycolic acid, the monomethacrylates of glycol, glycerol, and of other polyhydric alcohols, the monomethacrylates of dialkylene glycols and polyalkylene glycols. The corresponding acrylates in each instance may be substituted for the methacrylates. Examples include the following: 2-hydroxyethyl acrylate or methacrylate, diethylene glycol acrylate or methacrylate, 2-hydroxypropyl acrylate or methacrylate, 3-hydroxypropyl acrylate or methacrylate, tetraethyleneglycol acrylate or methacrylate, pentaethyleneglycol acrylate or methacrylate, dipropyleneglycol acrylate or methacrylate, acrylamide, methacrylamide, diacetone acrylamide methylolacrylamide methylolmethacrylanide and any acrylate or methacrylate having one or more straight or branched chain alkyl groups of 1 to 30 carbon atoms, preferably 5 to 18 carbon atoms. Other suitable examples include isobornyl methacrylate, phenoxyethyl methacrylate, isodecyl methacrylate, stearyl methacrylate, hydroxypropyl methacrylate, cyclonexyl methacrylate, dimethylaminoethyl methacrylate, t-butylaminoethyl methacrylate, 2-acrylamido propane sulfonic acid, 2-ethylexyl methacrylate, methyl methacrylate, ethyl methacrylate, butyl methacrylate, 2-hydroxyethyl methacrylate, tetrahydrofurfuryl methacrylate and methoxyethyl methacrylate.

Examples of monounsaturated monomers containing carboxylic acid groups as functional groups and suitable for use as starting materials in accordance with the invention include the following: acrylic acid, methacrylic acid, itaconic acid, aconitic acid, cinnamic acid, crotonic acid, mesaconic acid, maleic acid, fumaric acid.

Partial esters of the above acids are also suitable as monosaturated monomers for use in accordance with the invention. Instances of such esters include the following: mono-2-hydroxypropyl aconitate, mono-2-hydroxyethyl maleate, mono-2-hydroxypropyl fumarate, mono-ethyl itaconate, monomethyl cellosolve ester of itaconic acid, monomethyl cellosolve ester of maleic acid.

Instances of suitable monounsaturated monomers containing amino groups as functional groups include the following: diethylaminoethyl acrylate or methacrylate, dimethylaminoethyl acrylate or methacrylate, monoethylaminoethyl acrylate or methacrylate, tert-butylaminoethyl methacrylate, para-amino styrene, ortho-amino styrene, 2-amino-4-vinyl toluene, piperidinoethyl methacrylate, morpholinoethyl methacrylate, 2-vinyl pyridine, 3-vinyl pyridine, 4-vinyl pyridine, 2-ethyl-5-vinyl pyridine, dimethylaminopropyl acrylate and methacrylate, dimethylaminoethyl vinyl ether, dimethylaminoethyl vinyl sulfide, diethylaminoethyl vinyl ether, amonoethyl vinyl ether, 2-pyrrolidinoethyl methacrylate, 3-dimethylaminoethyl-2-hydroxy-propylacrylate or methacrylate, 2-aminoethyl acrylate or methacrylate, isopropyl methacrylamide, N-methyl acrylamide or methacrylamide, 2-hydroxyethyl acrylamide or methacrylamide, 1-methacryloyl-2-hydroxy-3-trimethyl ammonium chloride or sulfomethylate, 2-(1-aziridinyl)-ethyl methacrylate. Polyethylenically unsaturated monomers which ordinarily act as though they have only one unsaturated group, such as isopropene, butadiene and chloroprene, should not be calculated as part of the polyunsaturated monomer content, but as part of the monoethylenically unsaturated monomer content.

4

In one embodiment, two polymerized polyunsaturated monomers are used. Allyl methacrylates polymerized with ethylene glycol dimethacrylate are particularly suitable for trapping a peroxide to form the peroxide composite. In such an embodiment, the diunsaturated monomers also function as cross-linking agents. This specific polymerization product is a polymeric porous sphere that appears as a powder.

In one embodiment, the polymer particulates useful herein are made by a process comprising:

(a) dissolving at least one and preferably at least two polyunsaturated monomers, preferably along with an effective amount of an organic polymerization initiator, in a water-immiscible silicone fluid solvent, to provide a monomer mixture;
(b) slowly agitating the dissolved monomers and silicone solvent;
(c) continuing slow agitation during polymerization of the monomers in the silicone fluid to produce microporous polymer micro-particles and agglomerates thereof;
and
(d) separating the microporous polymer micro-particles and agglomerates from the silicone solvent to produce microporous, polymer micro-particles and aggregates in the form of spheres.
In various embodiments, the sphere aggregates and sphere agglomerates having a diameter of less than about 500 microns, preferably less than about 100 microns, more preferably less than about 80 microns.

Porous cross-linked polymers among those useful herein are disclosed in U.S. Patents 5,955,552; and 6,387,995.
Such polymers include those commercially available as: MICROSPONGE® 5640, marketed by A.P. Pharma, Redwood City, California, U.S.A.; POLYTRAP® 6603 and POLY-PORE® 200 series, marketed by Amcol International Corp, Arlington Heights, Illinois, U.S.A.; and DSPCS®-12 series and SPCAT®-12 series, marketed by Kobo Products, Inc., South Plainfield, New Jersey, U.S.A.

The present invention provides compositions comprising an orally acceptable carrier. Selection of specific carrier components is dependant on the desired product form, such as dentifrices, rinses, powders, gels, confectionaries and paints. In various embodiments, it facilitates the adherence of a peroxide composite against surfaces within the oral cavity to which the composition is administered, without concomitant use of a dental tray or similar appliance. However, if the carrier is operable for use with a tape, tray, mouthpiece or similar appliance.

Materials among those that are useful in carriers include adhesion agents, viscosity modifiers, diluents, surfactants, foam modulators, peroxide activators, peroxide stability agents, abrasives, pH modifying agents, humectants, mouth feel agents, sweeteners, flavorants, colorants, and combinations thereof.

The carrier may include an adhesion agent, such as a material or combination of materials that enhance the retention of the peroxide composite on an oral cavity surface. Such adhesion agents may include adhesives, film forming materials, viscosity enhancers, and combinations thereof. Exemplary materials include hydrophilic organic polymers, hydrophobic organic polymers, silicone gums, silicas, and combinations thereof. Accordingly, the present invention provides oral care compositions comprising:

(a) a peroxide composite comprising a peroxide compound and a porous cross-linked polymer; and
(b) a non-aqueous carrier comprising an adhesion agent, preferably selected from the group consisting of adhesives, film forming materials, viscosity enhancers and combinations thereof, preferably comprising a material selected from the group consisting of hydrophilic organic polymers, hydrophobic organic polymers, silicone gums, silicas, and combinations thereof.
In one embodiment, the adhesion agent comprises a film forming material.

Hydrophilic organic polymers useful herein include polyethylene glycols, nonionic polymers of ethylene oxide, block copolymers of ethylene oxide and propylene oxide, carboxymethylene polymers, N-vinyl heterocyclic polymers, and mixtures thereof. Nonaqueous hydrophilic polymers useful in the practice of the present invention preferably provide a viscosity for the composition in the range between about 10,000 cps to 600,000 cps.

Hydrophilic polymers also include polymers of polyethylene glycols and ethylene oxide having the general formula:

$$HOCH_2(CH_2OCH_2)_nOH$$

wherein n represents the average number of oxyethylene groups. Polyethylene glycols available from Dow Chemical (Midland, Michigan, U.S.A.) are designated by number such as 200, 300, 400, 600, 2000 which represents the approximate average molecular weight of the polymer. Polyethylene glycols 200, 300, 400 and 600 are clear viscous liquids at room temperature, and are preferred for use in the practice of the present invention.

Another hydrophilic polymer useful herein is comprised of a water soluble, nonionic block copolymer of ethylene oxide and propylene oxide of the formula: $HO(C_2H_4O)_a(C_3H_6O)_b(C_2H_4O)_cH$. The block copolymer is preferably chosen (with respect to a, b and c) such that the ethylene oxide constituent comprises from about 65 to about 75% by weight, of the copolymer molecule and the copolymer has an average molecular weight of from about 2,000 to about 15,000, with the copolymer being present in oral care composition in such concentration that the composition is liquid at room temperature (23°C).

A block copolymer useful herein is Pluraflo L1220, which has an average molecular weight of about 9,800. The hydrophilic poly(ethylene oxide) block averages about 65% by weight of the polymer.

Organic polymers useful as adhesion enhancing agents include hydrophilic polymers such as carbomers such as carboxymethylene polymers such as acrylic acid polymers, and acrylic acid copolymers. Carboxypolymethylene is a slightly acidic vinyl polymer with active carboxyl groups. One such polymer is CARBOPOL® 974 marketed by Noveon, Inc., Cleveland, Ohio, U.S.A..

N-vinyl heterocyclic polymers useful herein include those derived from a N-heterocyclic vinyl monomer, preferably comprising N-vinyl heterocyclic monomers having from 3 to 7 atoms in a heterocyclic ring, including a carbonyl carbon atom and a nitrogen heteroatom containing a vinyl group. Preferably the ring contains 5 or 6 atoms, comprises heteroatoms such as sulfur or oxygen, and may be substituted or unsubstituted. Certain embodiments are the polymers of specific N-vinyl heterocyclic monomers such as N-vinyl imides to form poly-N-vinyl polyimides, and N-vinyl lactams to form poly-N-vinyl polylactams, and mixtures thereof. In a preferred embodiment, the polymer is poly-N-vinyl-poly-2-pyrrolidone. The poly-N-vinyl-poly-2-pyrrolidone is also commonly known as polyvinylpyrrolidone or "PVP". The polymers include soluble and insoluble homopolymeric PVPs. Copolymers containing PVP include vinylpyrrolidone/vinyl acetate (also known as Copolyvidone, Copolyvidonum or VP-VAc) and vinylpyrrolidone/dimethylaminoethylmethacrylate. Soluble PVP polymers among those useful herein are known in the art, including Povidone, Polyvidone, Polyvidonum, poly (N-vinyl-2-pyrrolidinone), poly (N-vinylbutyrolactam), poly(1-vinyl-2-pyrrolidone) and poly [1-(2-oxo-lpyrrolidinyl)ethylene]. These PVP polymers are not substantially cross-linked. In various embodiments of this invention, an insoluble cross-linked homopolymer is preferred. Such polymers include those commonly referred to in the art as polyvinylpolypyrrolidone, cross-povidone, and cPVP, and are referred to herein as "cPVP."

Hydrophobic organic materials useful as adhesion enhancing agents in the practice of the present invention include hydrophobic materials such as waxes such as bees wax, mineral oil, mineral oil and polyethylene blends, petrolatum, white petrolatum, liquid paraffin, butane/ethylene/styrene hydrogenated copolymer) blends (VERSAGEL® marketed by Penreco, Houston, Texas, U.S.A.), acrylate and vinyl acetate polymers and copolymers, polyethylene waxes, silicone polymers as discussed further herein and polyvinyl pyrrolidone/vinyl acetate copolymers. In embodiments of the present invention containing a hydrophobic polymer, present in ratios of about 1 to about 85% weight of the composition.

Silicone polymers useful herein include, but are not limited to, silicone adhesives, silicone elastomers, silicone fluids, silicone resins, silicone gums and mixtures thereof. In one embodiment, the carrier comprises a pressure sensitive adhesive (PSA) composition, including those that are well known in the art. Generally, silicone based PSA's are produced by condensing a silicone resin and an organosiloxane such as a polydiorganosilioxane. Suitable silicone polymers include the copolymer product of mixing a silanol terminated polydiorganosiloxane such as polydimethyl siloxane with a silanol-containing silicone resin whereby the silanol groups of the polydiorganosiloxane undergo a condensation reaction with the silanol groups of the silicone resin so that the polydiorganosiloxane is lightly crosslinked by the silicone resin (that is, the polydiorganosiloxane chains are bonded together through the resin molecules to give chain branching and entanglement and/or a small amount of network character) to form the silicone pressure sensitive adhesive. A catalyst (for example, an alkaline material such as ammonia, ammonium hydroxide or ammonium carbonate) can be mixed with the silanol-terminated polydiorganosiloxane and the silicone resin to promote this crosslinking reaction. Copolymerizing the silicone resin with the silanol terminated polydiorganosiloxane affords a self adhering and cohesive soft elastomer matrix. Modifying the silicone resin to polydiorganosiloxane ratio of the pressure sensitive adhesive will modify the tackiness of the oral care composition. For example PSAs are available from the Dow-Corning Corporation, Midland, Michigan, U.S.A., under the brand name BIO-PSA. The silicone based pressure sensitive adhesive is present in the liquid whitening compositions of the present invention at a concentration of about 0.5% to about 99%.

Silicone gums useful herein include high molecular weight polydiorganosiloxanes having a viscosity at 25° C of from about 500,000 cS up to about 50,000,000 cS. Such silicone gums include those polydiorganosiloxanes with an average molecular weight of greater than 500,000. The polysiloxane gums for use herein can be linear or cyclic, and branched or unbranched. Substituents may have any structure as long as the resulting polysiloxanes are hydrophobic, are not irritating, toxic or otherwise harmful when applied to the oral cavity, and are compatible with the other components of the composition, Specific examples of siloxane gums include polydimethylsiloxane, methylvinylsiloxane, copolymer, poly(dimethylsiloxane, diphenyl, methyvinylsiloxane) copolymer, and mixtures thereof.

Polysiloxane fluids useful herein include those with a viscosity, at 25° C, of from about 1 cS to about 1000 cS, optionally from about 2 cS to about 500 cS, or from about 5 cS to about 400 cS. Polysiloxane fluids for use herein can be linear or cyclic, and can be substituted with a wide variety of substituents (including as described above). Preferred substituents include methyl, ethyl and phenyl substituents. Suitable polysiloxane fluids include linear polysiloxane polymers such as dimethicone and other low viscosity analogues of the polysiloxane materials, preferably having a viscosity at 25°C. of 200 cS or less and cyclomethicone, and other cyclic siloxanes having for example a viscosity at 25° C. of 200 cS or less.

Adhesion agents also include inorganic materials. Such inorganic materials include silicon polymers such as amorphous silica compounds which function as thickening agents (CAB-O-SIL® fumed silica manufactured by Cabot Corporation, Boston, Massachusetts, U.S.A.; and SYLOX® 15 also known as SYLODENT® 15, marketed by Davison Chemical Division of W.R. Grace & Co., Columbia, Maryland, U.S.A.). A preferred adhesion agent is Dow Coming Silicone Adhesive 8-7016, marketed by Dow-Coming Corporation.

Thickening agents among those useful herein include carboxyvinyl polymers, carrageenans (also known as Irish moss and more particularly iota-carrageenan), cellulosic polymers such as hydroxyethylcellulose, carboxymethylcellulose (carmellose) and salts thereof (e.g., carmellose sodium), natural gums such as karaya, xanthan, gum arabic and tragacanth, colloidal magnesium aluminum silicate, colloidal silica, and mixtures thereof. One or more thickening agents are optionally present in a total amount of about 0.01 % to about 15%, for example about 0.1% to about 10% or about 0.2% to about 5% by weight of the composition.

Viscosity modifiers among those useful herein include mineral oil, petrolatum, clays and organomodified clays, silica, and mixtures thereof. In various embodiments, such viscosity modifiers are operable to inhibit settling or separation of ingredients or to promote redispersibility upon agitation of a liquid composition. One or more viscosity modifiers are optionally present in a total amount of about 0.01% to about 10%, for example about 0.1% to about 5% by weight of the composition.

Diluents among those useful herein include materials or combinations of materials that are operable to solubilize and/or suspend other components of the composition. In various embodiments, diluents are operable to adjust the viscosity of the composition, optionally in conjunction with viscosity modifiers (as discussed herein) and other components of the composition. In various embodiments, the composition is non-aqueous, i.e., does not contain appreciable amounts of chemically-unbound water. Diluents include glycerin and anhydrous alcohol. Diluents are present in the nonaqueous liquid whitening compositions of the present invention in amounts of about 0.1% to about 90%, optionally in various embodiments about 0.5% to about 70%, about 0.5% to about 50%, about 0.5% to about 35%.

Surfactants among those useful herein include anionic, nonionic, and amphoteric surfactants. Surfactants may be used, for example, to provide enhanced stability of the formulation, to help in cleaning the oral cavity surfaces through detergency, and to provide foam upon agitation, e.g., during brushing with a dentifrice composition of the invention. Suitable anionic surfactants include water-soluble salts of $C_{8-20}$ alkyl sulfates, sulfonated monoglycerides of $C_{8-20}$ fatty acids, sarcosinates, taurates. Illustrative examples of these and other surfactants are sodium lauryl sulfate, sodium coconut monoglyceride sulfonate, sodium lauryl sarcosinate, sodium lauryl isoethionate, sodium laureth carboxylate and sodium dodecyl benzenesulfonate, and mixtures thereof. Suitable nonionic surfactants include poloxamers, polyoxyethylene sorbitan esters, fatty alcohol ethoxylates, alkylphenol ethoxylates, tertiary amine oxides, tertiary phosphine oxides, dialkyl sulfoxides. Suitable amphoteric surfactants include derivatives of $C_{8-20}$ aliphatic secondary and tertiary amines having an anionic group such as carboxylate, sulfate, sulfonate, phosphate or phosphonate. A suitable example is cocoamidopropyl betaine. One or more surfactants are optionally present in a total amount of about 0.01% to about 10%, for example about 0.05% to about 5% or about 0.1% to about 2%. Preferably, the surfactant is nonionic and compatible with peroxide compounds such as polyethylene oxide. Nonionic surfactants are present in embodiments of this invention at levels of about 0.01% to about 1%.

Foam modulators useful herein include materials operable to increase amount, thickness or stability of foam generated by the composition (e.g., dentifrice compositions) upon agitation. Any orally acceptable foam modulator can be used, including polyethylene glycols (PEGs), also known as polyoxyethylenes. High molecular weight PEGs are suitable, including those having an average molecular weight of about 200,000 to about 7,000,000, for example about 500,000 to about 5,000,000 or about 1,000,000 to about 2,500,000. One or more PEGs are optionally present in a total amount of about 0.1% to about 10%, for example about 0.2% to about 5% or about 0.25% to about 2%.

Humectants useful herein include polyhydric alcohols such as glycerin, sorbitol, xylitol or low molecular weight PEGs. In various embodiments, humectants are operable to prevent hardening of paste or gel compositions upon exposure to air. In various embodiments humectants also function as sweeteners. One or more humectants are optionally present in a total amount of about 1% to about 50%, for example about 2% to about 25% or about 5% to about 15%.

Peroxide activators such as sodium bicarbonate, sodium carbonate, manganese gluconate may be incorporated in the compositions of the present invention. The activator is relatively nonactive with the peroxide whitening agent in nonaqueous liquid compositions.

pH modifying agents among those useful herein include acidifying agents to lower pH, basifying agents to raise pH, and buffering agents to control pH within a desired range. Any orally acceptable pH modifying agent can be used, including without limitation carboxylic, phosphoric and sulfonic acids, acid salts (e.g., monosodium citrate, disodium citrate, monosodium malate), alkali metal hydroxides such as sodium hydroxide, carbonates such as sodium carbonate, bicarbonates, sesquicarbonates, borates, silicates, phosphates (e.g., monosodium phosphate, trisodium phosphate, pyrophosphate salts), imidazole, and mixtures thereof. One or more pH modifying agents are optionally present in a total amount effective to maintain the composition in an orally acceptable pH range.

Mouth-feel agents include materials which impart a desirable texture or other feeling during use of the composition. Such agents include bicarbonate salts, and orally acceptable bicarbonate can be used, including without limitation alkali metal bicarbonates such as sodium and potassium bicarbonates, ammonium bicarbonate, and mixtures thereof. One or more bicarbonate salts are optionally present in a total amount of 0.1% to about 50%, for example about 1% to about 20%.

Flavorants among those useful herein include any material or mixture of materials operable to enhance the taste of the composition. Any orally acceptable natural or synthetic flavorant can be used, such as flavoring oils, flavoring aldehydes, esters, alcohols, and combinations thereof. Also encompassed within flavorants are ingredients that provide fragrance and/or other sensory effect in the mouth, including cooling or warming effects. Such ingredients include menthol, menthyl acetate, menthyl lactate, camphor, eucalyptus oil, eucalyptol, anethole, eugenol, cassia, oxanone, $\alpha$-irisone, propenyl guaiethol, thymol, linalool, benzaldehyde, cinnamaldehyde, N-ethyl-p-menthan-3-carboxamine, N,2,3-trimethyl-2-isopropylbutanamide, 3-1-menthoxypropane-1,2-diol, cinnamaldehyde glycerol acetal (CGA), methone glycerol acetal (MGA), and mixtures thereof.

Sweeteners among those useful herein include orally acceptable natural or artificial, nutritive or non-nutritive sweeteners. Such sweeteners include dextrose, polydextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, sucralose, dipeptide-based intense sweeteners, cyclamates, dihydrochalcones, and mixtures thereof.

Colorants among those useful herein include pigments, dyes, lakes and agents imparting a particular luster or reflectivity such as pearling agents. Any orally acceptable colorant can be used, including talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride, and mixtures thereof.

In one embodiment, the present invention provides compositions comprising:

(a) about 0.5% to about 60% of a peroxide composite; and
(b) about 0.5% to about 60% of a silicone adhesive.
Optionally, the composition additionally comprises about 1 to about 99% of a silicone fluid. Optionally, the composition additionally comprises from about 1% to about 40% of a hydrophobic polymer, such as a blend of mineral oil and polyethylene glycol.

The compositions of the present invention optionally comprise an abrasive. In various embodiments, an abrasive is useful for example as a polishing agent. Any orally acceptable abrasive can be used, but type, fineness (particle size) and amount of abrasive should be selected so that tooth enamel is not excessively abraded in normal use of the composition. Suitable abrasives include silica, for example in the form of silica gel, hydrated silica or precipitated silica, alumina, insoluble phosphates, calcium carbonate, resinous abrasives such as urea-formaldehyde condensation products, and mixtures thereof. Among insoluble phosphates useful as abrasives are orthophosphates, polymetaphosphates and pyrophosphates. Illustrative examples are dicalcium orthophosphate dihydrate, calcium pyrophosphate, $\beta$-calcium pyrophosphate, tricalcium phosphate, calcium polymetaphosphate and insoluble sodium polymetaphosphate.

The compositions of the present invention optionally may include any other ingredient active or inert, desirable for use in a specific oral composition, such as tartar control agents, anti-caries agents, fluoride ion sources, stannous ion sources, zinc ion sources, zinc ion sources, anti-inflammatory agents, antibacterial agents, antioxidants sialogues, breath freshening agents, antiplaque agents, bacterial anti-adhesion agents, desensitizing agents, enzymes and anticalculus agents.

The compositions of the present invention optionally comprise a fluoride ion source useful, for example, as an anti-caries agent. Any orally acceptable fluoride ion source can be used, including potassium, sodium and ammonium fluorides and monofluorophosphates, stannous fluoride, indium fluoride, amine fluorides, such as olaflur (N'-octadecyltrimethylendiamine-N,N,N'-tris(2-ethanol)-dihydrofluoride), and mixtures thereof. In various embodiments, water-soluble fluoride ion sources are used. One or more fluoride ion sources are optionally present in an amount providing a total of about 0.0025% to about 2%, for example about 0.005% to about 1% or about 0.01% to about 0.3%, of fluoride ions.

The compositions of the present invention are made by any of a variety of methods, including by adding and mixing the ingredients of the composition in a suitable vessel such as a stainless steel tank provided with a mixer. In one embodiment, the composition components are added to the mixing vessel in the following order: liquid ingredients, thickener ingredients, peroxide composite and any flavoring, colorant or sweetener. While the exact mixing order is not determinate, it some embodiments it is preferred that the peroxide composite be one of the last components added in the multicomponent composition. The ingredients are then mixed to form a homogeneous dispersion/solution.

The present invention provides methods for whitening a tooth surface using compositions according to the present invention. As referred to herein, "tooth" or "teeth" refers to natural teeth, dentures, dental plates, fillings, caps, crowns, bridges, dental implants, and any other hard surfaced dental prosthesis either permanently or temporarily fixed within the oral cavity. As used herein, "whitening" refers to a change in visual appearance of a tooth, preferably such that such that the tooth has an increase in whiteness. Increase in whiteness of a dental surface can be observed visually, for example with the aid of color comparison charts or gauges, or measured by colorimetry, using any suitable instrument such as a Minolta Chromameter, e.g., model CR-400 (Minolta Corp., Ramsey, NJ). The instrument can be programmed, for example, to measure Hunter Lab values or L*a*b* values according to the standard established by the International Committee of Illumination (CIE). The L*a*b* system provides a numerical representation of three-dimensional color space where L* represents a lightness axis, a* represents a red-green axis and b* represents a yellow-blue axis. The L* and b* axes are typically of greatest applicability to measurement of tooth whiteness. Increase in whiteness can be computed from differences in L*, a* and b* values before and after treatment, or between untreated and treated surfaces. A useful parameter is ΔE*, calculated as the square root of the sum of the squares of differences in L*, a* and b* values, using the formula:

$$\Delta E^* = [(\Delta L^*)2 + (\Delta a^*)2 + (\Delta b^*)2]1/2$$

A higher value of ΔE* indicates greater increase in whiteness. In various embodiments, the method of the present invention can effect a ΔE* of at least about 1, or at least about 3, or at least about 5.

Accordingly, the present invention provides a method for whitening a tooth surface according to claim 16. Preferably the method comprises applying a composition of the present invention, comprising the peroxide composite and a carrier. As referred to herein, "applying" refers to any method by which the peroxide composite is placed in contact with the tooth surface. Such methods, in various embodiments, comprise direct application of a composition by such methods as rinsing, painting, and brushing. In various embodiments, application of the composition comprises the use of an application device which aids in maintaining contact of the composite to the tooth surface for sufficient time so as to allow whitening.

In one preferred embodiment, the whitening composition is applied using a "paint on" technique. A small application device, such as a brush or spatula is coated with a composition of this invention and the composition is then placed on a tooth surface. Preferably, the composition be spread evenly on such surfaces, in sufficient quantity to deliver a whitening amount of peroxide composite.

The present invention also provides methods for effecting the controlled release of an oxidizing peroxide species onto a tooth surface, comprising contacting the surface with a peroxide composite of the present invention. As referred to herein, such controlled release in various embodiments comprises sustained release of the peroxide composite. Without limiting the mechanism, function or utility of present invention, in some embodiments contact with saliva causes the slow release of peroxide active from the cross-linked polymer matrix to the applied tooth site. The slow release provides enhanced whitening by providing a constant supply of an effective amount of peroxide over a prolonged period of time. Preferably, the composition is contacted with the tooth surface for at least about 30 seconds, optionally at least about 1 minute.

The present invention is further illustrated through the following nonlimiting examples.

Example 1

A composition of the present invention is made by combining the ingredients shown in Table I, below:

Table I

| Ingredients | Weight % |
|---|---|
| POLYPORE® E200, micronized - 85.7% Hydrogen Peroxide | 14.00 |
| COP CARBOPOL® 974 P | 2.00 |
| Polyethylene Glycol - PEG2M | 83.00 |
| Poly Ethylene Oxide - 99.5% | 1.00 |

(continued)

| Ingredients | Weight % |
|---|---|
| Total | 100.00 |

The composition is applied to the teeth of a human subject having stained teeth, resulting in perceptible whitening. The composition is determined to provide a ΔE value of about 5.

Example 2

A composition of the present invention is made by combining the ingredients show in Table II, below:

Table II

| Ingredients | Weight % |
|---|---|
| POLYPORE® E200, micronized with 85.7% Hydrogen peroxide | 15.00 |
| Vinyl Acetate Copolymer (PLASDONE® - 630) | 0.50 |
| PLURAFLOW® (alkoxylate surfactant) L4370 (BASF) | 79.70 |
| Silica | 3.50 |
| Anhydrous alcohol | 0.50 |
| Saccharin | 0.80 |
| Total | 100.00 |

Example 3

Compositions of the present invention are made by combining the ingredients of Table III, below

Table III

| Ingredients | Weight % | | |
|---|---|---|---|
| | Formula 1 | Formula 2 | Formula 3 |
| POLY-PORE® E200, micronized with 85.7% Hydrogen Peroxide | 14 | 18 | 23.3 |
| DC350 Medical Fluid | - | 10 | 10 |
| DC 8-7016 Fluid (Silicone Adhesive) | - | 30 | 30 |
| PLASTIGEL® 5 | - | 42 | 36.7 |
| COP CARBOPOL® 974 P | 2 | - | - |
| Polyethylene Glycol - PEG2M | 83 | - | - |
| Poly Ethylene Oxide - 99.5% | 1 | - | - |
| Total (%) | 100 | 100 | 100 |

The resulting composition is subjected to accelerated aging 40.5°C/48.9°C (105°F/120°F) for several weeks, and remains stable with more than 90% of the peroxide remaining. An *in vitro* hydrogen peroxide retention test is also conducted for the composition. The results of the test, set forth in the following table, show that the composition has a retention of hydrogen peroxide over time.

| Retention Time (minutes) | |
|---|---|
| | Retained $H_2O_2$ (%) |
| 0 | 100 |

(continued)

| Retention Time (minutes) | |
|---|---|
| | Retained $H2O_2$ (%) |
| 5 | - |
| 10 | 60 |
| 20 | 58 |
| 30 | 51 |
| 45 | 46 |
| 60 | 19 |

**Claims**

1. An oral care composition, comprising:

   (a) a peroxide composite comprising a peroxide compound and a porous cross-linked polymer wherein the cross-linked polymer has a BET pore volume of 0.1 to 0.3 cc/g, and the peroxide composite comprises the peroxide compound sorbed, at a level of from 70% to 90% by weight of the peroxide compound to the crosslinked polymer, wherein the porous cross-linked polymer comprises a polymerized polyunsaturated monomer selected from the group consisting of polyacrylates, polymethacrylates, polyitaconates, and mixtures thereof; and
   (b) an orally acceptable carrier.

2. An oral care composition according to claim 1, wherein the polyunsaturated monomer is a polymethacrylate.

3. An oral care composition according to claim 1, wherein the polymer comprises two of the polymerized polyunsaturated monomers.

4. An oral care composition according to claim 3, wherein the polymer comprises allyl methacrylates polymerized with ethylene glycol dimethacrylate.

5. An oral care composition according to claim 1, wherein the cross-linked polymer has a BET pore volume of 0.14 to 0.16 cc/g.

6. An oral care composition according to claim 1, wherein the peroxide compound comprises hydrogen peroxide.

7. An oral care composition according to claim 1, wherein the carrier is nonaqueous.

8. An oral care composition according to claim 7 , wherein the carrier comprises a material selected from the group consisting of adhesion agents, surfactants, peroxide activators, solvents, flavorants, sweeteners, colorants, and mixtures thereof.

9. An oral care composition according to claim 8, wherein the carrier comprises an adhesion agent selected from the group consisting of silicone polymers, waxes, mineral oil, petrolatum, hydrophilic polymers, silicas, and mixtures thereof.

10. An oral care composition according to claim 8 , wherein the carrier comprises a hydrophilic polymer selected from the group consisting of polyethylene glycols, nonionic polymers of ethylene oxide, block copolymers of ethylene oxide and propylene oxide, carboxymethylene polymers, polyvinyl pyrrolidone, and mixtures thereof.

11. An oral care composition according to claim 9, wherein the carrier comprises a silicone polymer selected from the group consisting of silicone adhesives, silicone elastomers, silicone fluids, silicone resins, silicone gums and mixtures thereof.

12. An oral care composition comprising:

(a) a peroxide composite comprising a peroxide compound and a porous cross-linked polymer wherein the peroxide composite comprises the peroxide compound sorbed, at a level of 70% to 90% by weight of the active, onto the crosslinked polymer, wherein the porous cross-linked polymer comprises a polymerized polyunsaturated monomer selected from the group consisting of polyacrylates, polymethacrylates, polyitaconates and mixtures thereof; and

(b) a non-aqueous carrier comprising a film forming material.

13. An oral care composition according to claim 12, wherein the film forming material comprises a hydrophilic polymer.

14. An oral care composition according to claim 12, wherein the carrier comprises a silicone.

15. An oral care composition according to claim 12, wherein the porous cross- linked polymer comprises a polymerized polyunsaturated monomer comprising a polymethacrylate.

16. A method for whitening a tooth surface, comprising applying to the surface a safe and effective amount of a peroxide composite comprising a peroxide compound and a porous cross-linked polymer, wherein the peroxide composite comprises the peroxide compound sorbed, at a level of 70% to 90% by weight of the active, onto the crosslinked polymer, wherein the porous cross-linked polymer comprises a polymerized polyunsaturated monomer selected from the group consisting of polyacrylates, polymethacrylates, polyitaconates and mixtures thereof.

17. A method according to claim 16, wherein the porous cross-linked polymer comprises a polymerized polyunsaturated monomer comprising a polymethacrylate.

18. A method according to claim 16, comprising applying the peroxide composite in a non-aqueous carrier additionally comprising a material selected from the group consisting of adhesion agents, surfactants, peroxide activators, solvents, flavorants, sweeteners, colorants, and mixtures thereof.

19. A method according to claim 18, wherein the carrier comprises an adhesion agent selected from the group consisting of silicone polymers, waxes, mineral oil, petrolatum, hydrophilic polymers, silicas, and mixtures thereof.

20. A method according to claim 18, wherein the carrier comprises a hydrophilic polymer selected from the group consisting of polyethylene glycols, nonionic polymers of ethylene oxide, block copolymers of ethylene oxide and propylene oxide, carboxymethylene polymers, polyvinyl pyrrolidone, and mixtures thereof.

21. A method according to claim 18, wherein the carrier comprises a silicone polymer selected from the group consisting of silicone adhesives, silicone elastomers, silicone fluids, silicone resins, silicone gums and mixtures thereof.

**Patentansprüche**

1. Mundhygienezusammensetzung, umfassend:

(a) ein Peroxid-Komposit, das eine Peroxid-Verbindung und ein poröses, vernetztes Polymer umfasst, wobei das vernetzte Polymer ein BET Porenvolumen von 0,1 bis 0,3 cc/g hat und im Peroxid-Komposit die Peroxid-Verbindung mit einem Gehalt von 70 Gew.% bis 90 Gew.% der Peroxid-Verbindung auf dem vernetztem Polymer sorbiert ist und wobei das poröse, vernetzte Polymer ein polymerisiertes, mehrfach ungesättigtes Monomer ausgewählt aus der Gruppe bestehend aus Polyacrylaten, Polymethacrylaten, Polyitaconaten und Mischungen davon umfasst; und

(b) einen oral annehmbaren Träger.

2. Mundhygienezusammensetzung nach Anspruch 1, wobei das mehrfach ungesättigte Monomer ein Polymethacrylat ist.

3. Mundhygienezusammensetzung nach Anspruch 1, wobei das Polymer zwei der polymerisierten, mehrfach ungesättigten Monomere umfasst.

4. Mundhygienezusammensetzung nach Anspruch 3, wobei das Polymer Allylmethacrylate umfasst, die mit Ethylenglykoldimethacrylat polymerisiert sind.

5.  Mundhygienezusammensetzung nach Anspruch 1, wobei das vernetzte Polymer ein BET Porenvolumen von 0,14 bis 0,16 cc/g hat.

6.  Mundhygienezusammensetzung nach Anspruch 1, wobei die Peroxid-Verbindung Wasserstoffperoxid umfasst.

7.  Mundhygienezusammensetzung nach Anspruch 1, wobei der Träger wasserfrei ist.

8.  Mundhygienezusammensetzung nach Anspruch 7, wobei der Träger ein Material umfasst, das aus der Gruppe bestehend aus Adhäsionsmitteln, Tensiden, Peroxidaktivatoren, Lösungsmitteln, Geschmackstoffen, Süßungsmitteln, Farbstoffen und Mischungen davon ausgewählt ist.

9.  Mundhygienezusammensetzung nach Anspruch 8, wobei der Träger ein Adhäsionsmittel umfasst, das aus der Gruppe bestehend aus Silikonpolymeren, Wachsen, Mineralöl, Vaseline, hydrophilen Polymeren, Kieselgelen und Mischungen davon ausgewählt ist.

10. Mundhygienezusammensetzung nach Anspruch 8, wobei der Träger ein hydrophiles Polymer umfasst, das aus der Gruppe bestehend aus Polyethylenglykolen, nichtionischen Polymeren von Ethylenoxid, Block-Copolymeren von Ethylenoxid und Propylenoxid, Carboxymethylenpolymeren, Polyvinylpyrrolidon und Mischungen davon ausgewählt ist.

11. Mundhygienezusammensetzung nach Anspruch 9, wobei der Träger ein Silikonpolymer umfasst, das aus der Gruppe bestehend aus Silikonadhesiven, Silikonelastomeren, Silikonflüssigkeiten, Silikonharzen, Silikongummis und Mischungen davon ausgewählt ist.

12. Mundhygienezusammensetzung umfassend:

    (a) ein Peroxid-Komposit, das eine Peroxid-Verbindung und ein poröses, vernetztes Polymer umfasst, wobei im Peroxid-Komposit die Peroxid-Verbindung mit einem Gehalt von 70 Gew.% bis 90 Gew.% der Peroxid-Verbindung auf dem vernetztem Polymer sorbiert ist und wobei das poröse, vernetzte Polymer ein polymerisiertes, mehrfach ungesättigtes Monomer ausgewählt aus der Gruppe bestehend aus Polyacrylaten, Polymethacrylaten, Polyitaconaten und Mischungen davon umfasst; und
    (b) einen wasserfreien Träger, der ein filmbildendes Material umfasst.

13. Mundhygienezusammensetzung nach Anspruch 12, wobei das filmbildende Material ein hydrophiles Polymer umfasst.

14. Mundhygienezusammensetzung nach Anspruch 12, wobei der Träger ein Silikon umfasst.

15. Mundhygienezusammensetzung nach Anspruch 12, wobei das poröse, vernetzte Polymer ein polymerisiertes, ungesättigtes Monomer umfasst, das ein Polymethacrylat umfasst.

16. Verfahren zur Aufhellung einer Zahnoberfläche, bei dem eine sichere und effektive Menge eines Peroxid-Komposits und eines porösen, vernetzten Polymers auf die Zahnoberfläche aufgetragen wird, wobei im Peroxid-Komposit die Peroxid-Verbindung mit einem Gehalt von 70 Gew.% bis 90 Gew.% der Peroxid-Verbindung auf dem vernetzten Polymer sorbiert ist und wobei das poröse, vernetzte Polymer ein polymerisiertes, mehrfach ungesättigtes Monomer umfasst, das aus der Gruppe bestehend aus Polyacrylaten, Polymethacrylaten, Polyitaconaten und Mischungen davon ausgewählt ist.

17. Verfahren nach Anspruch 16, bei dem das poröse, vernetzte Polymer ein polymerisiertes, mehrfach ungesättigtes Monomer umfasst, das Polymethacrylat umfasst.

18. Verfahren nach Anspruch 16, bei dem das Peroxid-Komposit in einem wasserfreien Träger aufgetragen wird, der zusätzlich ein Material umfasst, das aus der Gruppe bestehend aus Adhäsionsmitteln, Tensiden, Peroxidaktivatoren, Lösungsmitteln, Geschmackstoffen, Süßungsmitteln, Farbstoffen und Mischungen davon ausgewählt ist.

19. Verfahren nach Anspruch 18, bei dem der Träger ein Adhäsionsmittel umfasst, das aus der Gruppe bestehend aus Silikonpolymeren, Wachsen, Mineralöl, Vaseline, hydrophilen Polymeren, Kieselgelen und Mischungen davon ausgewählt ist.

**20.** Verfahren nach Anspruch 18, bei dem der Träger ein hydrophiles Polymer umfasst, das aus der Gruppe bestehend aus Polyethylenglykolen, nichtionischen Polymeren von Ethylenoxid, Block-Copolymeren von Ethylenoxid und Propylenoxid, Carboxymethylenpolymeren, Polyvinylpyrrolidon und Mischungen davon ausgewählt ist.

**21.** Verfahren nach Anspruch 18, bei dem der Träger ein Silikonpolymer umfasst, das aus der Gruppe bestehend aus Silikonadhesiven, Silikonelastomeren, Silikonflüssigkeiten, Silikonharzen, Silikongummis und Mischungen davon ausgewählt ist.

## Revendications

**1.** Composition d'hygiène orale comprenant :

(a) un composite de peroxyde comprenant un composé peroxyde et un polymère réticulé poreux, dans lequel le polymère réticulé a un volume de pores BET de 0,1 à 0,3 cm$^3$/g, le composite de peroxyde comprenant le composé peroxyde sorbé, en une proportion de 70 % à 90 % en poids du composé peroxyde sur le polymère réticulé, dans lequel le polymère réticulé poreux comprend un monomère polyinsaturé polymérisé choisi dans l'ensemble constitué par les polyacrylates, les polyméthacrylates, les polyitaconates, et leurs mélanges ; et
(b) un véhicule acceptable par voie orale.

**2.** Composition d'hygiène orale selon la revendication 1, dans laquelle le monomère polyinsaturé est un polyméthacrylate.

**3.** Composition d'hygiène orale selon la revendication 1, dans laquelle le polymère comprend deux des monomères polyinsaturés polymérisés.

**4.** Composition d'hygiène orale selon la revendication 3, dans laquelle le polymère comprend des méthacrylates d'allyle polymérisés avec du diméthacrylate d'éthylèneglycol.

**5.** Composition d'hygiène orale selon la revendication 1, dans laquelle le polymère réticulé a un volume de pores BET de 0,14 à 0,16 cm$^3$/g.

**6.** Composition d'hygiène orale selon la revendication 1, dans laquelle le composé peroxyde comprend du peroxyde d'hydrogène.

**7.** Composition d'hygiène orale selon la revendication 1, dans laquelle le véhicule est non aqueux.

**8.** Composition d'hygiène orale selon la revendication 7, dans laquelle le véhicule comprend un matériau choisi dans l'ensemble constitué par les agents d'adhérence, les tensioactifs, les activateurs de peroxyde, les solvants, les agents de saveur, les édulcorants, les colorants, et leurs mélanges.

**9.** Composition d'hygiène orale selon la revendication 8, dans laquelle le véhicule comprend un agent d'adhérence choisi dans l'ensemble constitué par les polymères siliconés, les cires, l'huile minérale, le pétrolatum, les polymères hydrophiles, les silices, et leurs mélanges.

**10.** Composition d'hygiène orale selon la revendication 8, dans laquelle le véhicule comprend un polymère hydrophile choisi dans l'ensemble constitué par les polyéthylèneglycols, les polymères non-ioniques d'oxyde d'éthylène, les copolymères séquencés d'oxyde d'éthylène et d'oxyde de propylène, les polymères de carboxyméthylène, la polyvinylpyrrolidone, et leurs mélanges.

**11.** Composition d'hygiène orale selon la revendication 9, dans laquelle le véhicule comprend un polymère siliconé choisi dans l'ensemble constitué par les adhésifs siliconés, les élastomères siliconés, les fluides siliconés, les résines siliconées, les gommes siliconées, et leurs mélanges.

**12.** Composition d'hygiène orale comprenant :

(a) un composite de peroxyde comprenant un composé peroxyde et un polymère réticulé poreux, dans lequel le composite de peroxyde comprend le composé peroxyde sorbé, en une proportion de 70 % à 90 % en poids de l'agent actif, sur le polymère réticulé, dans lequel le polymère réticulé poreux comprend un monomère polyinsaturé polymérisé choisi dans l'ensemble constitué par les polyacrylates, les polyméthacrylates, les polyitaconates, et leurs mélanges ; et

(b) un véhicule non aqueux comprenant un matériau filmogène.

13. Composition d'hygiène orale selon la revendication 12, dans laquelle le matériau filmogène comprend un polymère hydrophile.

14. Composition d'hygiène orale selon la revendication 12, dans laquelle le véhicule comprend une silicone.

15. Composition d'hygiène orale selon la revendication 12, dans laquelle le polymère réticulé poreux comprend un monomère polyinsaturé polymérisé comprenant un polyméthacrylate.

16. Procédé pour blanchir une surface dentaire, comprenant l'application sur la surface d'une quantité efficace et sans danger d'un composite de peroxyde comprenant un composé peroxyde et un polymère réticulé poreux, dans lequel le composite de peroxyde comprend le composé peroxyde sorbé, en une proportion de 70 % à 90 % en poids de l'agent actif, sur le polymère réticulé, dans lequel le polymère réticulé poreux comprend un monomère polyinsaturé polymérisé choisi dans l'ensemble constitué par les polyacrylates, les polyméthacrylates, les polyitaconates, et leurs mélanges.

17. Procédé selon la revendication 16, dans lequel le polymère réticulé poreux comprend un monomère polyinsaturé polymérisé comprenant un polyméthacrylate.

18. Procédé selon la revendication 16, comprenant l'application du composite peroxyde dans un véhicule non aqueux comprenant de plus un matériau choisi dans l'ensemble constitué par les agents d'adhérence, les tensioactifs, les activateurs de peroxyde, les solvants, les agents de saveur, les édulcorants, les colorants, et leurs mélanges.

19. Procédé selon la revendication 18, dans lequel le véhicule comprend un agent d'adhérence choisi dans l'ensemble constitué par les polymères siliconés, les cires, l'huile minérale, le pétrolatum, les polymères hydrophiles, les silices, et leurs mélanges.

20. Procédé selon la revendication 18, dans lequel le véhicule comprend un polymère hydrophile choisi dans l'ensemble constitué par les polyéthylèneglycols, les polymères non-ioniques d'oxyde d'éthylène, les copolymères séquencés d'oxyde d'éthylène et d'oxyde de propylène, les polymères de carboxyméthylène, la polyvinylpyrrolidone, et leurs mélanges.

21. Procédé selon la revendication 18, dans lequel le véhicule comprend un polymère siliconé choisi dans l'ensemble constitué par les adhésifs siliconés, les élastomères siliconés, les fluides siliconés, les résines siliconées, les gommes siliconées, et leurs mélanges.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9107184 A
- US 20040086468 A
- WO 2005070378 A
- WO 2006026424 A
- US 3759880 A
- US 3992562 A
- US 4013825 A
- US 4276402 A
- US 4341889 A
- US 4224427 A
- US 4250322 A
- US 4423099 A
- US 4543398 A
- US 4136250 A
- US 5955552 A
- US 6387995 A